Europäisches Patentamt

European Patent Office

Office européen des brevets

(19)

(11) Publication number: **0 246 110 B1**

(12) EUROPEAN PATENT SPECIFICATION

(45) Date of publication of patent specification: **31.07.91**

(51) Int. Cl.⁵: **A61M 5/14**, F16K 7/06

(21) Application number: **87304336.8**

(22) Date of filing: **15.05.87**

(54) Valve for controlling the flow of liquid through a tube.

(30) Priority: **16.05.86 GB 8611980**

(43) Date of publication of application:
**19.11.87 Bulletin 87/47**

(45) Publication of the grant of the patent:
**31.07.91 Bulletin 91/31**

(84) Designated Contracting States:
**DE FR GB SE**

(56) References cited:
**US-A- 3 497 175**
**US-A- 3 550 861**
**US-A- 3 759 483**

(73) Proprietor: **Juhasz, Laszlo**
**Woodhead 51 Kilmardinny Avenue**
**Bearsden Glasgow G61 3NL Scotland(GB)**

(72) Inventor: **Juhasz, Laszlo**
**Woodhead 51 Kilmardinny Avenue**
**Bearsden Glasgow G61 3NL Scotland(GB)**

(74) Representative: **Perry, Robert Edward et al**
**GILL JENNINGS & EVERY 53-64 Chancery**
**Lane**
**London WC2A 1HN(GB)**

## Description

This invention relates to valves which can be used to control the flow of fluid, i.e. liquid or gas, through a tube.

Such control is required, for example, generally in the irrigation of a wound caused by injury or operation, and specifically in continuous ambulatory peritoneal dialysis (CAPD). It is conventional to control the irrigation flow in-line, by breaking the tube, and inserting a stopcock, or by applying a clamp to the tube (whose walls are of a deformable material).

CAPD and its requirements are described in WO-A-8301572. In general terms, each patient has four cycles of treatment per day; in each cycle, an on/off valve has to be used four times. There are thus 16 switching actions per day. Three primary requirements of a CAPD patient for any online flow valve are (1) easy and simple operation, because a large proportion of patients are elderly or invalid; (2) robust construction which can stand up to a minimum of 1 year's safe usage, i.e. at least 5800 manipulations; and (3) small physical size, without sharp edges or corners, because the valve is part of the fluid transfer system which is permanently attached to the body of the patient.

For example, an online stopcock can be used to control liquid flow, but wears badly, depending on the material from which its 3-4 parts are contructed and the nature of the liquid (the material and the liquid are directly in contact, and the material is subject to contamination and corrosion). Such stopcocks tend to leak. Their action is undefined.

Online one-piece spring clamps are known, having a number of settings by which fluid flow through the tube can be controlled. Their short-term performance is acceptable, but long-term performance is poor owing to the ageing of the material. Such clamps are often of irregular shape, with edges which limit their clinical acceptability.

Other clamps are known, in which a roller can be moved along a guide within a housing, from a flow-constricting position to a non-constricting position at opposite ends of the housing. Such roller clamps can wear well and exhibit good performance even in long-term operation, subject to the nature of the material from which they are constructed. However, their action is poorly defined, intermediate positions and thus the effect of the roller on fluid flow being unrepeatable, and the irregular shape of such clamps limits their clinical acceptability. Such valves are used in the Travenol Ambu-flex (registered Trade Mark) CAPD system.

US-A-3550861 discloses a hose nozzle including a valve adapted to control the flow of liquid through a flexible tube, the valve comprising mutually-engaging essentially cylindrical male and female members, in which the male member has three apertures in its side-wall, and in which the internal diameter of the female member varies gradually from relatively narrow to relatively broad; and three balls, acting as a flow-constricting member, which are constrained to pass inwardly through the apertures when in contact with the relatively narrow internal diameter of the female member.

According to the present invention, in an on-line valve of the general type described immediately above (although there will usually be only a single flow-constricting member and corresponding aperture), the valve includes positive location means, comprising a groove in the relatively narrow internal diameter portion of the female mamber for the flow-constricting member at maximum flow constriction (zero flow).

In one embodiment of the invention, the male and female members engage frictionally, and flow control (non-specific) is caused by push-pull operation.

In a second embodiment of the invention, the male and female members have mutually-engaging screw threads. By turning the male and female members relative to one another, the second embodiment of the invention allows fully-controllable, positive and accurate flow control. For this purpose, a calibration dial may be provided.

The invention will now be described by way of example only with reference to the accompanying drawings, in which:

Figures 1A and 1B are cross-sections along an axial plane of the same (first) embodiment of the invention, respectively showing the flow-constricting member in closed and open positions; and

Figures 2A and 2B are views analogous to those of Figs. 1A and 1B, respectively, but of the second embodiment of the invention.

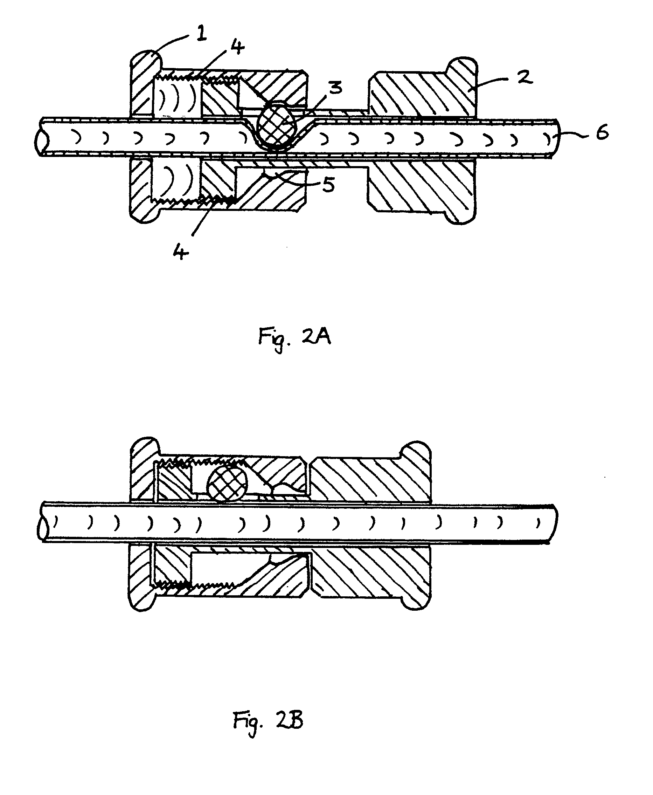

In the drawings, the female member is indicated as 1, the male member as 2 and the flow-constricting member as 3. Further, Figs. 2A and 2B show mutually-engaging screw threads 4 on the members 1 and 2. In each case, the members 1 and 2 can be displaced axially with respect to each other.

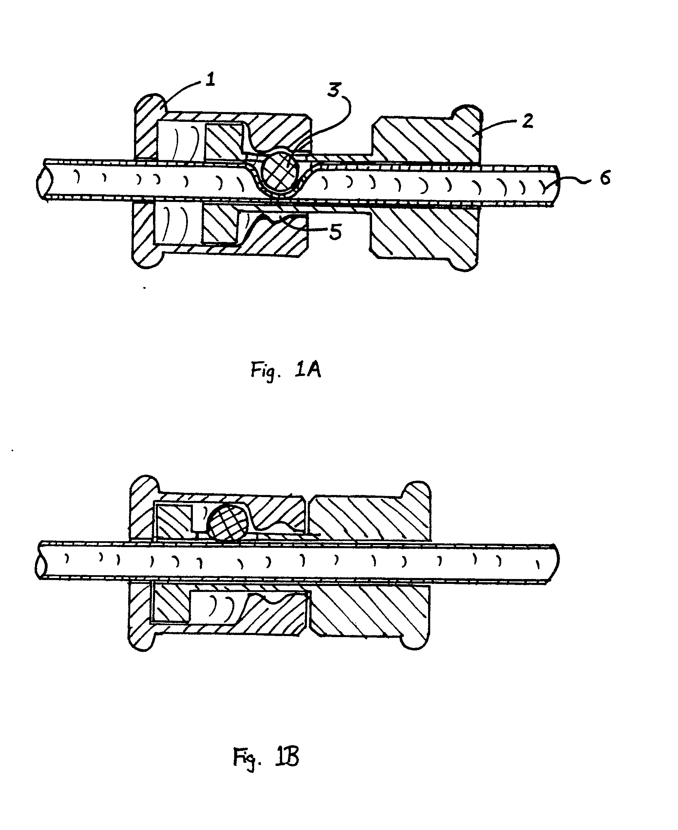

The drawings also show flexible tubing 6 which sits within the axial channel within the male member. The tubing 6 is respectively constricted and unconstricted in each of the two pairs of drawings. In the former case, (Figs. 1A and 2A), the flow-constricting member 3 is in contact with a relatively narrow internal diameter of the female member. A groove 5 provides means for positive location of the member 3 at zero flow. In the latter case, the member 3 is in contact with a relatively broad internal diameter.

The male and female members may be constructed of, for example, the same or different plastics materials, or of glass or metal. The flow-constricting member may be spherical (as illustrated in the drawings), e.g. a steel ball. The dimensions of the male and female members and the flow-constricting member can easily be defined so that the flow-constricting member is retained by the two members, at least when they are mutually-engaging.

As shown in the drawings, the female member includes a section of varying internal diameter, between the limits of the relatively narrow and relatively broad internal diameters. The section of variation allows liquid flow through the tubing to be controlled very easily, between maximum and nil rates.

Valves of the invention are simple to construct and use. On/off switching can be rapid. They do not suffer from the other disadvantages associated with the use of clamps. They can allow continuous variation of flow rates. They can be maintained in position on tubing as long as necessary, or moved along the tube when there is maximum flow. They can be operated manually or automatically (hydraulically, pneumatically or electrically).

There is no fluid/valve contact, in a valve of the invention. There are no wearable or ageing moving parts. The design is compact, without sharp edges or breakable parts.

Valves of the invention can be used in the medical field, generally for blood and intravenous administration systems and also, for example, for haemodialysis and peritoneal dialysis. In industry, they can be used for various chemicals, and also in the domestic field for food, drink, oil and gas. Generally and also in the scientific field, they are suitable as general purpose/continuously-variable online flow switches/controllers. The only limitation on their performance lies in the nature of the flexible tubing material.

## Claims

1. An on-line valve for controlling the flow of a fluid through a flexible tube (6), comprising:

   mutually-engaging essentially cylindrical male and female members (2,1), in which the male member (2) has an aperture in its sidewall, and in which the internal diameter of the female member (1) varies gradually from relatively narrow to relatively broad; and

   a flow-constricting member (3) which is constrained to pass inwardly through the aperture when in contact with the relatively narrow internal diameter of the female member (2),

   wherein, when the male and female members are engaged, they are axially moveable relative to each other, and define a bore in which the tube (6) can be located such that the flow-constricting member (3) is in contact with the tube (6);

   characterised by positive location means, comprising a groove (5) in the relatively narrow internal diameter portion of the female member (1) for the flow-constricting member (3) at maximum flow constriction (zero flow).

2. A valve according to claim 1, in which the male member (2) is a frictional fit within the female member (1), such that flow control is achieved by push-pull operation.

3. A valve according to claim 1, in which the male and female members (2,1) have mutually-engaging screw threads, which allows continuous variation in flow control.

4. A valve according to claim 3, which includes calibration.

5. A valve according to any preceding claim, which comprises a single flow-constricting member (3).

6. A combination of a valve according to any preceding claim and a flexible tube (6) located in the bore of the valve for use in the treatment of the human or animal body by surgery or therapy or in diagnostic methods practised on the human or animal body.

7. A combination according to claim 6, for use in the treatment of a wound by irrigation.

8. A combination according to claim 6, for use in blood administration, intravenous drug administration, peritoneal dialysis or haemodialysis.

9. A combination according to claim 8 for use in continuous ambulatory peritoneal dialysis.

## Revendications

1. Dispositif d'obturation en ligne destiné à commander l'écoulement d'un fluide au travers d'un tube flexible (6), comprenant :

   des éléments mâle et femelle essentiellement cylindrique s'engageant mutuellement (2, 1), dans lesquels l'élément mâle (2) possède une ouverture dans sa paroi latérale, et dans lesquels le diamètre interne de l'élément femelle (1) varie progressivement de relativement étroit vers relativement large; et

   un élément d'étranglement de l'écoulement (3) qui est contraint de passer en direction de l'intérieur au travers d'une ouverture lorsqu'il est en contact avec le diamètre interne relativement étroit de l'élément femelle (2),

dans lequel, lorsque les éléments mâle et femelle sont engagés, ceux-ci sont axialement mobiles l'un par rapport à l'autre et définissent un alésage dans lequel le tube (6) peut être positionné de telle sorte que l'élément d'étranglement de l'écoulement (3) est en contact avec le tube (6);

caractérisé par des moyens de positionnement positifs, comportant une gorge (5) dans la partie de diamètre interne relativement étroit de l'élément femelle (1) pour l'élément d'étranglement de l'écoulement (3) au niveau de l'étranglement d'écoulement maximum (écoulement nul).

2. Dispositif d'obturation selon la revendication 1, dans lequel l'élément mâle (2) est en engagement de friction à l'intérieur de l'élément femelle (1) de telle sorte que la commande de l'écoulement est réalisée par une opération poussertirer.

3. Dispositif d'obturation selon la revendication 1, dans lequel les éléments mâle et femelle (2, 1) ont des filets de vis s'engageant mutuellement, ce qui permet une variation continue de la commande de l'écoulement.

4. Dispositif d'obturation selon la revendication 3, qui comprend un étalonnage.

5. Dispositif d'obturation selon l'une quelconque des revendications précédentes, qui comprend un élément d'étranglement de l'écoulement (3) unique.

6. Combinaison d'un dispositif d'obturation selon l'une quelconque des revendications précédentes et d'un tube flexible (6) situé dans l'alésage de la dispositif d'obturation destinée à être utilisée dans le traitement du corps humain ou animal en chirurgie ou thérapie ou dans des méthodes de diagnostic pratiquées sur le corps humain ou animal

7. Combinaison selon la revendication 6, destinée à être utilisée dans le traitement d'une blessure par irrigation.

8. Combinaison selon la revendication 6, destinée à être utilisée dans l'administration de sang, l'administration de médicament intraveineuse, la dialyse péritonéale ou l'hémodialyse.

9. Combinaison selon la revendication 8, destinée à être utilisée dans la dialyse péritonéale ambulatoire continue.

## Patentansprüche

1. Leitungsventil zum Steuern eines Medienstroms durch ein biegsames Rohr (6) mit einem Patrizenteil (2) und einem Matrizenteil (1), die ineinandergesteckt und im wesentlichen zylindrisch sind, wobei das Patrizenteil (2) in seiner Seitenwand eine Öffnung hat und der Innendurchmesser des Matrizenteils (1) sich graduell von verhältnismäßig eng zu verhältnismäßig breit ändert, mit einem Absperrstück (3), das durch die Öffnung nach innen hindurchtritt, wenn es sich mit dem verhältnismäßig engen Durchmesser des Matrizenteils (2) in Kontakt befindet, wobei in der Eingriffslage beider Teile diese relativ zueinander achsial verschiebbar sind und eine Bohrung definieren, in die das Rohr (6) derart eingesetzt werden kann, daß das Absperrstück (3) am Rohr (6) anliegt, **dadurch gekennzeichnet,** daß im maximal abgesperrten Zustand (Strömung = Null) ein positives Lokalisierungsmittel mit einer Rille (5) für das Absperrstück (3) in dem Teil des Matrizenteils (1) mit dem relativ engen Innendurchmesser vorgesehen ist.

2. Leitungsventil nach Anspruch 1, **dadurch gekennzeichnet,** daß das Patrizenteil (2) im Matrizenteil (1) unter Reibschluß gehalten ist, so daß die Steuerung durch eine Zieh-Schieb-Bewegung erreicht wird.

3. Leitungsventil nach Anspruch 1, **dadurch gekennzeichnet,** daß die Teile (1, 2) über Gewinde miteinander verbunden sind, so daß die Strömung kontinuierlich gesteuert werden kann.

4. Leitungsventil nach Anspruch 3, **dadurch gekennzeichnet,** daß eine Kalibrierung vorgesehen ist.

5. Leitungsventil nach einem der Ansprüche 1 - 4, **dadurch gekennzeichnet,** daß ein einziges Absperrstück (3) vorgesehen ist.

6. Verwendung des Leitungsventils nach einem der Ansprüche 1 - 5 mit einem biegsamen Rohr (6) in der Bohrung des Ventils zur Behandlung des menschlichen oder tierischen Körpers bei Operationen, bei der Therapie oder bei der Diagnostik.

7. Kombination nach Anspruch 6 zur Verwendung bei der Wundbehandlung mittels Befeuchtung.

8. Kombination nach Anspruch 6 zur Verwendung bei der Blutzufuhr, intravenösen Arzneimittelgabe, Peritonealdialyse oder Hämodialyse.

9. Kombination nach Anspruch 8 zur Verwendung bei fortdauernder, ambulanter Peritonealdialyse.

Fig. 1A

Fig. 1B

Fig. 2A

Fig. 2B